# EUROPEAN PATENT SPECIFICATION

(11) **EP 0 408 933 B1**
(45) Date of publication and mention of the grant of the patent: **19.10.1994**
(21) Application number: 90112043.6
(22) Date of filing: 25.06.1990
(51) Int. Cl.: C12N 1/20, A23J 3/14, A61K 37/02, C12Q 1/68

(54) **A mutant of pseudomonas, a strain yzh, and a process for producing 851yzh nutrient solution by application of the strain**
Pseudomonasmutant, yzh-Stamm und Verfahren zur Herstellung der 851yzh-Nährlösung mittels dieses Stammes
Mutant de pseudomonas, souche yzh et procédé pour la préparation de la solution nutritive 851yzh par application de la souche

(30) Priority: 17.07.1989 CN 89104728
(43) Date of publication of application: 23.01.1991
(73) Proprietor: Yang, Zhenhua, Fu Zhou City Fu Jian Province (CN)
(72) Inventor: Yang, Zhenhua, Fu Zhou City Fu Jian Province (CN)
(74) Representative: Goddar, Heinz J., Dr.

(56) References cited:
- DE-A- 1 918 705
- US-A- 4 877 739
- PATENT ABSTRACTS OF JAPAN vol. 10, no. 36 (C-328), 13February 1986; & JP - A - 60188061
- KENKYUSHO) 25.09.1985
- WORLD PATENT INDEX Derwent Abstract no. 75-26654W [16], Derwent Publications Ltd., London, GB; &JP - A - 50006784

## Description

### FIELD OF THE INVENTION

The present invention relates to the production of single cell protein from plant protein by fermentation or incubation , using large scale industrial type equipment. The single cell protein is produced by the action of a mutant strain of pseudomonas on plant protein. The resulting product is a nutrient liquid or solution , or solid product made therefrom which is edible by human and animals.

### BACKGROUND OF THE INVENTION

Fermentation is an ancient and widely practiced art used to produce food and drink such as, sourdough bread, sauerkraut from cabbage, wine and beer. In some regions of the world where arable land is being increasingly used for non-food purposes, or where other food producing resources are looked for by means of the fermentation method. In the past , many microorganisms have been studied and attempts have been made to adapt them to industrial fermentation methods in order to produce proteins , in particular , single cell protein , which can be easily absorbed by the human body.

US Patent NO. 4 ,877 ,739 discloses a group of autogenic antiamonia azotobacter designed as 851 yellow that is mutanted from Azotobacter vinelandii , having the capability of antiamonia nitrofixation. This microorganism can also be used for manufacturing a single cell protein liquid enriched in Se , Zn, Ve, Vb, Vk, anticancer and autiaging tonic medicines. It also can be used for making bacteria manure , eel and animal forage, additive, antiseptic and binder.

Our invention discloses a process for manufacturing single cell protein from plant protein with a mutant strain of pseudomonas , or natural mutant or induced mutant of the said mutant strain. The resulting fermentation product , either a nutrient solution , a solid nutrient product or a product derived therefrom , is edible by human and animals. The nutrient solution of the present invention has the effects of antiaging, stimulating and improving hemogenesis in animals. protecting gastric mucosa of animals and human, especially, obviously inhibiting cancer cell growth in vitro and in vivo in animals and human, even reversing the cancer cells to their normal status.

### SUMMARY OF THE INVENTION

This invention provides a method for producing a single cell protein nutrient solution rich in more than twenty (20̸) amino acids as required by the human body and various trace elements by means of a mutant of pseudomonas , or natural mutant and induced mutant thereof, in the conventional industrial fermentation method. The strain of the microorganism of pseudomonas , designated as strain YZH , is particularly useful in manufacturing single cell protein from a soybean material , although it can also be used with other plant material such as starch , potatoes , sweet potatoes. Soybeans or soybean protein which has been incubated or fermented with strain 851 YZH according to the present invention is effectively converted into a single cell protein which is easily assimilated by the human body. Typically , this fermentation is carried out in aqueous medium to produce a single cell protein nutrient solution herein designated as 851 YZH nutrient solution or 851 YZH NS. As used herein , solution, liquid or nutrient solution means a true solution , or a suspension or mixture of a solid in a liquid to give a resulting solution - like fluid mass.

The microorganism of this invention is a mutant of pseudomonas designated as strain YZH , said pseudomonas having all the identifying characteristics of the sample on deposit with the American Type Culture Collection , 1230̸1 Parklawn Drive , Rockville , Maryland, 20̸852, and assigned A.T.C.C. Deposit NO. 53980̸.

This invention also relates to using the pseudomonas , strain YZH, in a conventional industrial fermentation process to produce a single cell protein nutrient solution comprising using pseudomonas YZH, or cultivated pseudomonas YZH as the single cell protein producing strain in a producing medium which may be a soybean , soybean derived protein or starch medium , or other media susceptible to the manufacture of single cell proteins. Addtionally , the invention is also related to effects of the nutrient solution on mammalian and human.

### THE BRIEF DESCRIPTION OF THE DRAWING

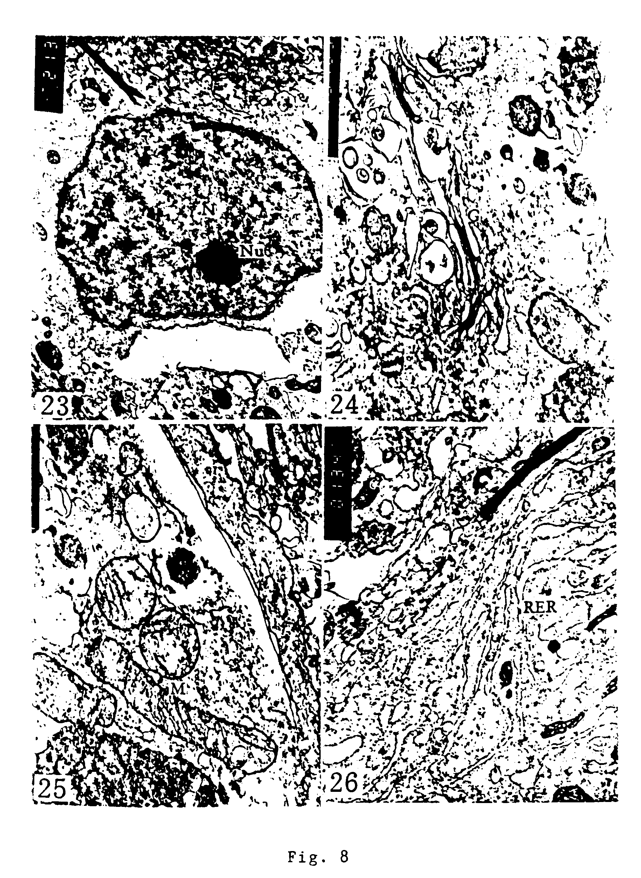

Fig. 1 is a diagram of the cellular growth of Human Gastric Adenocarcinoma Cell , MGC80̸-3 cell in vitro by the effect of 851 YZH nutrient solution , wherein the percentage is concentration of the solution.
Fig. 2 is a diagram of the cellular mitotic index of the MGC80̸-3 cells in vitro by the effect of 851 YZH nutrient solution.
Fig. 3 is a viewing of the MGC80̸-3 cells under phase contrast microscope.
Fig. 4 is a viewing of the MGC80̸-3 cells which are treated with 15% 851 YZH solution , stained with hematoxylin-Erosin (H.E ) under phase contrast microscope.
Fig. 5 is a scanning electron micrograph of the MGC80̸-3 cells
Fig. 6 is a scanning electron micrograph of the MGC80̸-3 cells which are treated with 20̸% 851 YZH nutrient solution.
Fig. 7 is a transmission electron micrograph of the MGC80̸-3 cell, wherein:
   - N:: nucleus
   - Nu:: nucleolus
   - G:: Gogli complex
   - M :: mitochondria
   - ER:: endoplasmic reticulum
Fig. 8 is a transmission electron micrograph of the MGC80̸-3 cells which are treated with 20̸% 851 YZH nutrient solution.

### THE DETAILED DESCRIPTION OF THE INVENTION

The microorganism of this invention is a mutant pseudomonas designated Pseudomonas YZH and having all the characteristics of the sample on deposit with the American Type Culture Collection and assigned A.T. C. C. Deposit NO. 53980̸. The strain YZH is found in and seperated from soybean or soybean derivated material. The characteristics of the strain YZH are that it is a blant rod in light yellow color , it has at least one flagellum, the thallus is in size of 0̸.5-0̸.7×1.2-2.0̸ um, it is a chemosynthetic heterotroph, it is unable to produce water-soluble pigment, it does not have a ability to form intracellular poly -β-hydroxy butyrate, and to produce arginine dihydrolase, it does not have an action of denitrification , it has the ability to reduce nitrates and to liquidize gelatine , it is obligately aerobic , the growth temperature is about 10̸-41°C , the perferable PH value is 4.5-9.0̸, the content of gaunine and cytosine in DNA of the strain is 65.4-67.7 mol% , and the DNA of the strain contains a Y fragment which can be detected by a special Y fragment DNA probe. The all features as mentioned above are the differential features of the YZH strain from the other gena of pseudomonas. When strain YZH is used in a nitrogen containing medium as taught in this invention, it can produce a single cell protein solution enriched in more than twenty (20̸) amino acids and a variety of trace elements and vitamins. Analysis of 10̸0̸ml samples of the nutrient solution produced by YZH is found to contain the following range of substances : 350̸-80̸0̸ mg of amino acids, 0̸.2 - 0̸.5 mg of Vitamin E , 0̸.0̸5 - 0̸.1 mg Vitamin B2 , 0̸.7 - 1.0̸ mg Vitamin PP or Niacin , 0̸.0̸4 - 0̸.0̸8 ppm Se , 0̸.2 - 0̸.5 ppm Zn , 0̸.8 - 0̸.24 ppm Mo, 0̸.0̸8 - 0̸.24 ppm Co, 0̸.0̸5 - 0̸.0̸6 ppm Mn, 0̸.0̸7 - 0̸.2 ppm Cu and 0̸.1 - 0̸.7 ppm Fe ; 1.21 - 1.38g dry substance , 1.21 - 2.2 g proteins. 0̸.11-0̸.13g lipids, and other substances.

The process for producing 851 YZH NS through the use of the YZH pseudomonas includes the following steps:
A culture medium for cultivating YZH is placed in a conventional fermentation tank which is equipped with a mixing means and gas inlet/outlet means so that the tank content can be homogenized and also aerated by the introduciton of filtered aspectic air needed by YZH and other attendent microorganisms during the period of culturing. The culture medium is soybeans or starch or other suitable materials as provided herein. It is seeded in a two-step processes using a first, second 851 YZH seed culture for 38-54 hours. After the seeding is completed , the resulting thallus is inoculated into a pre-autoclaved production medium of soybeans, starch or other suitble material for a third continuous culture or incubation. The production medium can contain other materials such as nutritionally important trace elements in addition to soybeans or starch as is described before. Once the thallus has been inoculated for about 24- 72 hours with filtered aspetic air passing through the mass. Incubation conditions are: aeration rate of 1 :0̸.6-1.2(mass/air) v/v min; mixing speed of 180̸-260̸ rpm; and the temperature of 28-38°C. After the incubation is finished, the resulting cultured broth is autoclaved and harvested. The broth ( nutrient solution , or liquid ) can be packaged directly to give a product suitble for human use. Alternative produces can also be obtained to fulfill a variety of needs by means of other processing. For example , the culture solution can be centrifuged and precipitated and then the filtrated by a thin membrane or super filtration to give a product which can be edible directly by human and animals.

Furthermore , the filtrated solution is further extracted with an organic solvents such as alochol , acetone ethylacetate or extracted with column extractor such as ion exchange column, wide-aperture resin column to give a final product , which contans a concentrated nutrition components. The culture solution can be added in any optional way into foods or drugs to produce a product containing 851 YZH pesudomona. The solution can also be dried to give a solid product , and the solution can be use as a replacement for water and eggs in the baking of bread , cakes and the like , or in other foods. The culture solution can also be used in cosmetic preparation.

In the method described above , the following production media have been used. Components are listed by weight with water making up the remainder. Proper amounts of trace elements necessary to the human body in addition to the these media are added. The benzoic acid or sodium benzoate can also be used in said media. The amounts of such elements are typically in the range of 1.5-10̸0̸ppm.

### 1. Soybean Medium

| | |
|---|---|
| Soybean | 5-10̸% |
| or Soybean milk or bean cake (by weight of soybean) | 5-15% |
| Yeast extract | 0̸.0̸2-0̸.5% |
| or yeast powder | 0̸.0̸2-0̸.5% |
| or peptone, beef extract | 0̸.0̸2-0̸.3% |
| CaCO₃ | 0̸.0̸5-0̸.25% |
| KH₂Po₄ | 0̸.0̸2-0̸.1% |
| MgSo₄ | 0̸.0̸1-0̸.0̸5% |
| NaCl | 0̸.0̸1-0̸.0̸4% |
| Na₂MoO₄ | 5.0̸ -30̸ppm |
| Na₂SeO₃ | 2.5 -15ppm |
| ZnSo₄ | 2.5 -40̸ppm |
| CoCl₂ | 5.0̸ -20̸ppm |

### 2. 1640̸ medium

The medium 1640̸ which is well known can be also used for the culture of YZH pseudomonas.

The 851 YZH nutrient solution , used as is , or in any of its forms, possesses salutory effects including promoting or protecting the health of the animals , especially inhibiting the growth of cancer cells in vivo and in vitro in animals and human. In order to more fully demonstrate the salutary effects of 851 YZH NS and it derivative, the following experiments and their results are presented.

### Experiment 1, Anti-lipid Peroxidative Effect

The oxygen free radical, hydroxy free radical and peroxide, especially lipid peroxide in the human and animals body are one of reasons of killing cells and causing the acute and chronic diseases and senility of human and animals. Some foods or drugs having an effect of direct or indirect anti-lipid peroxide may be able to provent from corcers or tumors and from senility of human and animals who do not have a enough ability of anti-peroxide or have a hyperactivity of lipid peroxide.(Harman, D Free Radicals in Mol, Biol, Aging & Diseases 1984, Raven Press, New York, pp1-12).

Kunming mice weighing 17.8 ± 1.75g were used and randomly divided into several groups (A.B.C ) of 10̸ mice each according to body weight. One of these group was the control group (A), all mice were given the same diet and mice of group (C) except the control group and group (B) was given 851 YZH nutrient solution for 13 consecutive days. Group A and B were given tap water. All mice were tasted at the 14th day. After tasting , mice of group (A ) was administrated paraffin oil (a dissolvent of CCl₄ ) at the 14th day, interperitoneally at about 4 p. m. The mice of group (B) and group (C) were administrated with CCl₄ intraperitoneally at the 14th day at 4 p. m. The amount of administration was 1.87m mol/kg.

On the 15th day , the animals were sacrificed and liver samples were prepared . Malonaldehyde (MDA) in the liver was analyzed according to the method of Hiroshi Ohkawa et al. ( Anal. Biochem , 95 :351 - 356 (1979 ) ). 1 ,1,3,3 - tetramethoxypropane (Japan TCI, E, P, Grade) was used as the standard for the analysis of lipid peroxide. MDA was spectrophotometrically determined in n mol/ g and the data were statistically analzsed (H±SD). The results were given below:

**Table 1**

| Group | No.of mice | MDA n mol/g liver | | P value | |
|---|---|---|---|---|---|
| | | | | compared to control group(A) | compared to CCl4 group (B) |
| control (A) | 10̸ | 119.5 | 27.2 | - | - |
| CCl₄ (B) | 10̸ | 1698.9 | 287.6 | <0̸.0̸0̸1 | - |
| CCl₄ 851YZH (C) | 9 | 1350̸.2 | 170̸.8 | <0̸.0̸0̸1 | <0̸.0̸1 |

The results indicate that 851 YZH NS possesses potent anti-liquid peroxide activity by substantially lowing the MDA level. This property might be useful in combating aging.

### Experiment 2 Effect of 851 YZH NS on the hemoglobin and leucocyte total and classification of WBC of new-born mice.

Twelve (12) pregnant Kunming mice were fed normal diet and tap water. After parturition , the twelve mice , with their respective newborn, were randomly divided into control group (A ) and an 851 YZH NS test group (B ). The above noted mice of control group A were maintained on normal diet and tap water , mice of group B , however , had their diet supplemented with 851 YZH NS during the suckling period and after weaning. The test duration ran for 15 days after weaning. At the end of the 15 day period , a blood sample of the newborn mice was taken from the eyeball of the newborn mice for analysis. The results were as follow:

**Table 2**

| Effect of 851 YZH NS on leucocyte total and hemoglubin | | | | | |
|---|---|---|---|---|---|
| Group | No. newborn | Hemoglubin (g.x±SD) | P ** | total WBC (X10̸ /mm X±SD) | P ** |
| control (A) | 34 | 10̸.0̸±1.2 | - | 5.373±1.0̸40̸ | - |
| 851 YZH (B) | 33 | 10̸.7±1.0̸ | <0̸.0̸1 | 6.279±1.910̸ | <0̸.0̸5 |

| | | | | | |
|---|---|---|---|---|---|
| * Compared with the control group, | | | | | |
| ** both test, compared with the control group | | | | | |

**Table 3**

| Effect of 851 YZH NS on classification of WBC | | | | | |
|---|---|---|---|---|---|
| Group | no. newborn | neutrohil (% x±SD) | P1* | Lymphocyte (% X±SD) | P2* |
| Control (A) | 34 | 58±10̸ | - | 42±9 | - |
| 851 YZH NS(B) | 33 | 41±11 | <0̸.0̸0̸1 | 59±11 | <0̸.0̸0̸1 |

| | | | | | |
|---|---|---|---|---|---|
| *compared with the control group, both test. | | | | | |

The above data indicate that the feeding of 851 YZH NS, which contains more than 20̸ amino acid , several different vitamins and various trace elements , results in an increase in lymphocytes in the newborn mice which can be take as a stimulation and improvement of a mouse's ability to produce these cells.

### Experiment 3 Therapeutic effect of 851 YZH NS on acute injury to the gastric mucosa of rats.

16 male and female Wistar rats , weighting 220̸±20̸g, were used in this experiment. These rats were randomly divided into control group (A) and 851 YZH NS group (B). Group A was fed a normal diet and given tap water. Group B had a diet containing 851 YZH NS , each of them had about 8ml of the solution a day. After being fed for 15 day , two groups of rats had nothing to eat for 14 hours. Each rat was given 2.5mg indomethacin, once daily, for two days. Two days later, the rats were sacrificed and their stomaches removed and washed and observed under the naked eyes.

6 rats in group A, 2 rats in group B had acute bleeding in the gastric mucosa , but bleeding of mice in group A were significantly more severe. The bleeding in gastric mucosa of 6 group A rats and 2 groups B were locally restricted as to being spotted , stripped or stretched. Diffure hemorrhaging of the gastric mucosa occured in one group A rat. However , whereas the group A showed 6 - 12 spot hemorrhages , the 851 YZH NS group showed only 2 - 5 spot hemorrhages. There results indicate that the adminstration of 851 YZH NS can significantly reduce gastric mucosal bleeding.

### Experiment 4 Effect of 851 YZH NS on the human chronic gastritis

20̸ patients with chronic gastritis were diagnosed with the pathological examination. They were 21 - 62 years old , 10̸ male and 10̸ female, and the all had obvious clinical symptoms.

The all patients were randomly divided into two groups , control group and test group , each group was 10̸ patients. The control group was administrated with vitamin C for 21 days. The test group was adminstrated orally with 851 YZH NS , three times a day , 80 ml once, for 3 weeks. The results were given below:

**Table 4**

| Symptoms | Control group (n=10̸) | | | 851 YZH NS Group (n=10̸) | | |
|---|---|---|---|---|---|---|
| | cure | relief | ratio | cure | relief | ratio |
| abdominal discomfirt | 1/3 | 2/8 | 38 | 4/9 | 3/9 | 78 |
| abdominal distension | - | 2/10̸ | 20̸ | 6/7 | - | 86 |
| acid regurgitation | - | 1/7 | 14 | 2/4 | - | 50̸ |
| belching | 1/9 | 2/9 | 33 | 3/8 | 2/8 | 63 |
| nausea & vomit | 1/4 | - | 25 | 4/5 | - | 80̸ |
| anorexia | 1/7 | 2/7 | 43 | 4/5 | 1/5 | 10̸0̸ |

As noted above , compared with the control group , 851 YZH NS can improve the clinical symptoms of stomach disorder and relieve chronic gastritis.

### Experiment 5 Effect of 851 YZH NS on cancer cell growth

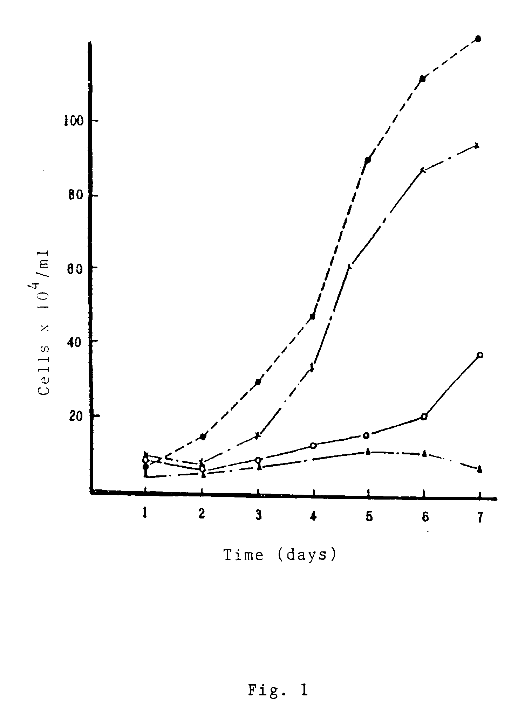

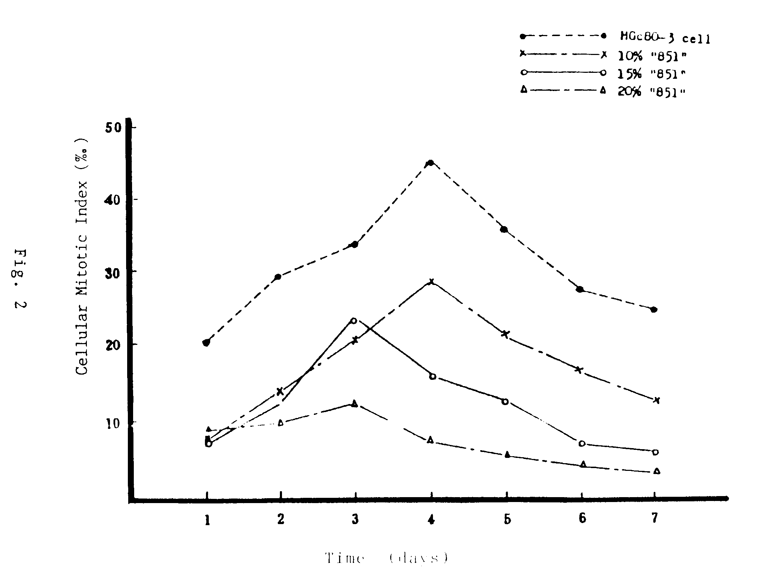

851 YZH NS of our invention possessed a strong inhibition of carcinoma cell growth , for example human gastric adenocarcinoma cells , MGC80̸-3 cells. Said cells were treated individually with 10̸% , 15% and 20̸% 851 YZH NS to give a good results. (see Fig. 1 and Fig. 2).

### Experiment 6 Effect of 851 YZH NS on human cancer cell observed under the electron microscope

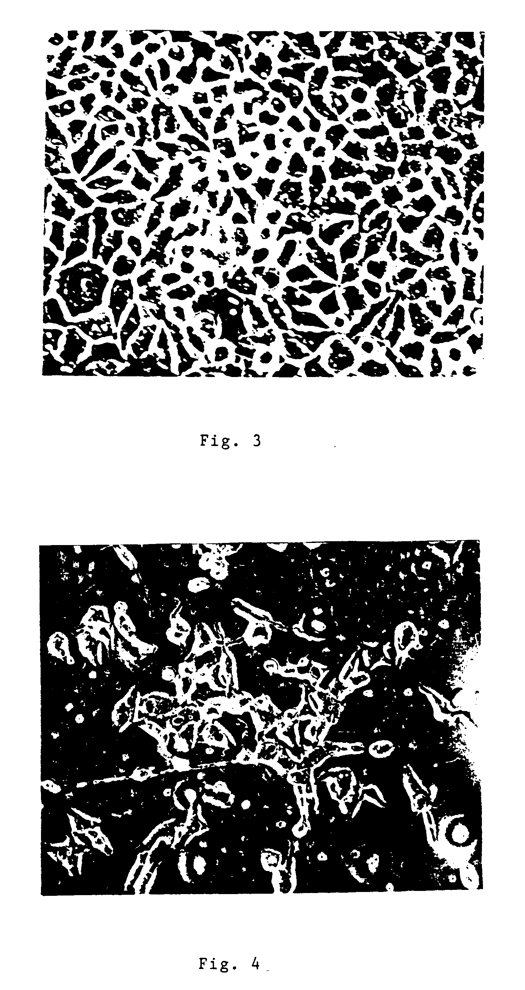

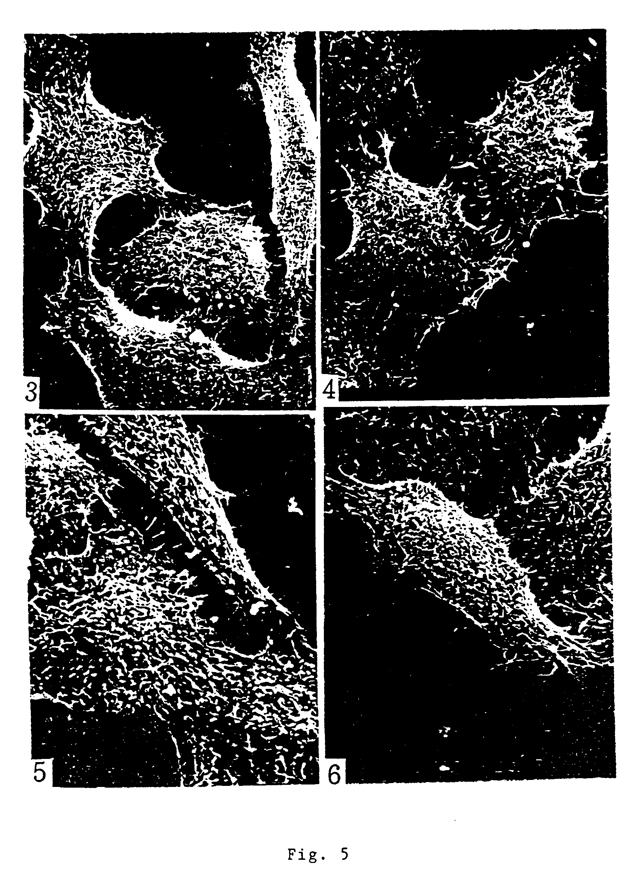

Human gastric adenocarcinoma , MGC80̸-3 cells, treated with 15% 851 YZH NS showed that there was an evident morphological change appeared in the cells stained with H. E. under phase contrast microscope. (see Fig. 2 and Fig. 3 ) In Fig. 4 and Fig. 5 scanning electron micrograph of the MGC80̸-3 cells showed that there were abundant filopodia existed at MGC 80̸- 3 cell's margin and microvilli densly covered their cells surface. But after the treatment of 20̸% 851 YZH NS , the microvilli at cells surface disappeared. Said cells had long cytoplasmic projection and lamellipodia at cell margin , and emerged with many winkle-like structures and blebs.

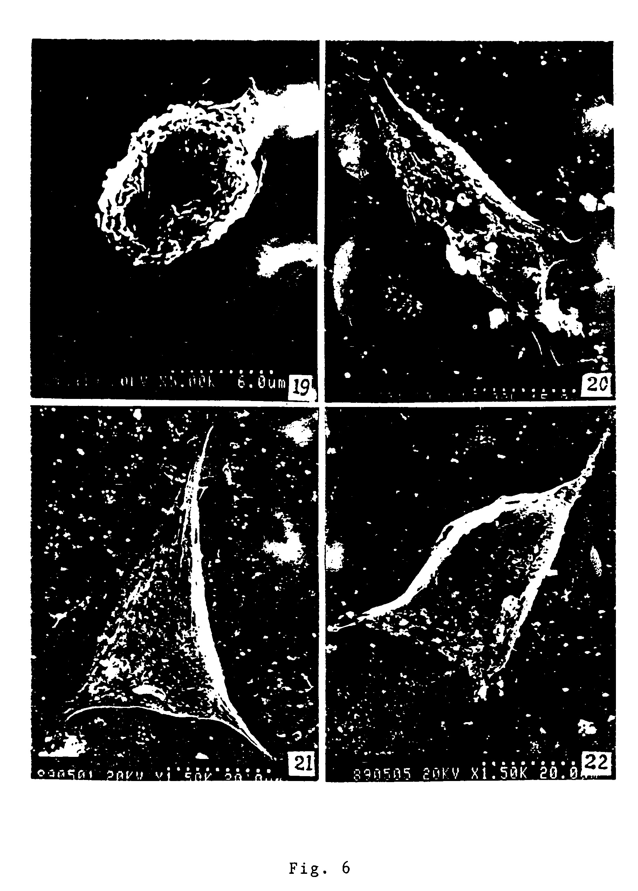

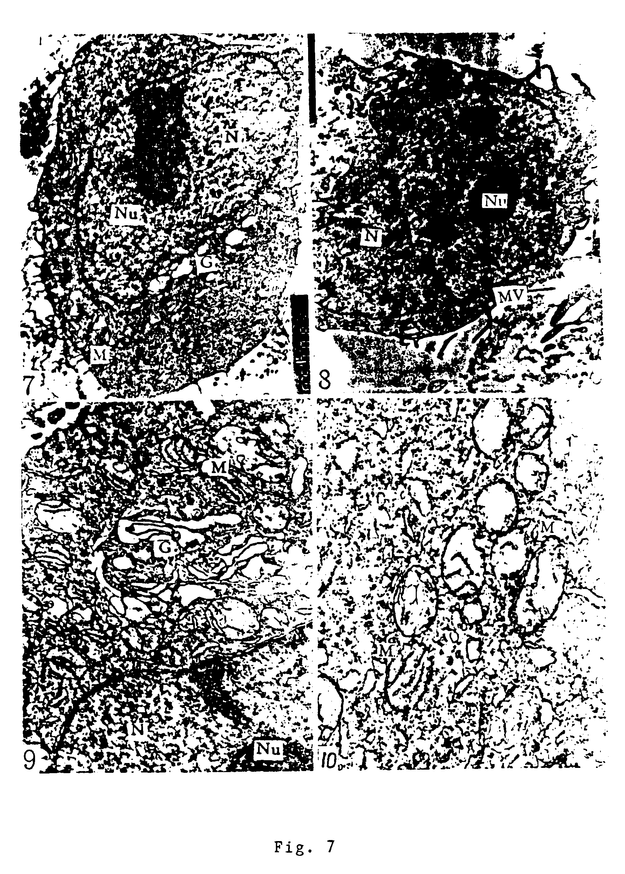

In Fig. 6 and Fig. 7, transmission electron micrograph showed that the MGC80̸- 3 cell was characterized with high nuclear - cytoplasmic ratio, irregular nuclear shape , large nucleolus , increasing heterochromatin and non-develpomental organelles in cytoplasm etc. After the treatment of 20% 851 YZH NS for about 7 - 9 days, the MGC80̸-3 cells had produced a series of ultrastructural changes similar to those of their corresponding normal cell , such as the nucleus (N ) was regular, neucleolus (Nu ) lessened , heterochromatin reduced and euchromatin increased , Golgi complex (G) and mitochondria (M) were developed and rough endoplasmic reticulum (RER ) increased as indicated in Fig. 7. The above results indicate that 851 YZH NS takes effect on inhibiting or killing the cancer cells in vitro , even on reversing the cancer cells into their normal cells. The inhibition and killing action on the cancer cells will take place at 12 - 24 hours in the treatment of said nutrient solution , Those cells that are not killed are cultured in the nutrient solution continousely for about 7 - 9 days. They can gradually change into their corresponding normal cells.

### Experiment 7 The result of mice heterotransplantation with the MGC80̸-3 cells and the cells treated with 15% 851 YZH NS

30̸ Kunming mice , weighing 17.5±1.65g , were randomly divided into three groups (A.B.C), each group had 10̸ mice.

The all mice of groups A ,B,C were given the MGC80̸-3 cells at the left forelegs. After feeding for 14 days , the mice were sacrificed. It was found that there were tumoriferous cases at the left foreleg armpits of all groups mice.

But , the mice of group A and C were administrated with the MGC80̸-3 cells that were treated with 15% NS for 3 days at the right forelegs. After the mice (20̸ ) were fed for 3 days. 10̸ mice had small tubercle at their right foreleg armpits.

In addition , the mice of groups C were given the MGC80̸-3 cells which were treated with 15% NS for 10̸ days, at their right forelegs. None of the mice had tumer.

The results indicate that 851 YZH NS has a strong inhibiting effect on cancer or tumor growth in animal body.

### Example 1

A 5 ton fermentation tank, equipped with mixing and aeration means was charged to 40̸% capacity. The fermentation medium was a 10̸% soybean medium consisting of , by weight , 20̸0̸ kg soybean that is processed by selecting , washing , milling and removing the residue , 80̸0̸g yeast extract , 10̸0̸0̸g KH₂PO₄ , 10̸0̸g MgSO₄ , 5kg CaCO₃ , 40̸0̸g NaCL, 20̸g Na₂MoO₄, 5gNa₂SeO₃, 20̸g ZnSO₄ , 20̸g CoCL₂ , 1kg Benzoic acid and water . The inoculum was 10̸% and consisted of a YZH pseudomonas cultured for 48 hours in a one-tenth weight of above mentioned medium in a 0̸.5 ton seed fermentation tank , which was charged with 20̸0̸ kilogram. The YZH pseudomonas was added in two stages as a first , second seed culture for about 48 hours. The inoculum was cultured at a temperature of 30̸°C , stirring speed of 260̸ rpm and aeration rate of 1:0̸.8 v/v min ( mass/ air ). After the seed culture was complete , the inoculum was added in the 5 ton fermentation tank for a third continuons inocubation. The fermentation medium and inoculum were allowed to react in the fermentation tank for a period of about 48 hours. The resulting broth (nutrient solution) was then autoclaved at 90̸°C and barometric pressure for a half hour, bottled on an assemble line and autoclaved again at an elevated temperature for a half hour to give the final product. Said final product , a solution , can be dried to give a solid product. Analysis of 10̸0̸ml of the broth showed it contained 2.2% dry material , 1.38% proteins , 0̸.13% lipids , 0̸.0̸9% carbohydrates, and vitamins, and minerals as is shown in Table 5.

**Table 5**

| Analytical report of 851 YZH NS | | | | | |
|---|---|---|---|---|---|
| Constituents | unit | Amount | Constituents | unit | Amount |
| eatable part | g | 10̸0̸ | | | |
| water | g | 97.8 | aspartate | mg | 66.5 |
| protein | g | 1.38 | glutamate | mg | 78.8 |
| lipids | g | 0̸.13 | serine | mg | 34.9 |
| carbohydrate | g | 0̸.0̸9 | proline | mg | 28.4 |
| heat capacity | Kcal | 7.1 | glycine | mg | 34.9 |
| eatable | | | alanine | mg | 41.2 |
| cellulose | g | - | threonine | mg | 31.5 |
| ash | g | 0̸.60̸ | valine | mg | 34.8 |
| - | - | - | methionine | mg | 15.0̸ |
| K | mg | 59.5 | isoleucine | mg | 24.0̸ |
| Na | mg | 7.7 | leucine | mg | 30̸.9 |
| Ca | mg | 4.9 | tyrosine | mg | 37.2 |
| Fe | mg | 0̸.6 | phenylalanine | mg | 28.5 |
| Zn | mg | 0̸.3 | lysine | mg | 18.5 |
| Cu | mg | 0̸.0̸7 | tryptophen | mg | 15.0̸ |
| Mn | mg | 0̸.0̸5 | histidine | mg | 17.1 |
| Mg | mg | 4.8 | arginine | mg | 42.0̸ |
| P | mg | 27.7 | - | - | - |
| Se | mg | 0̸.0̸4 | myristic acid | % | 7.1 |
| - | - | - | palmitic acid | % | 14.3 |
| vitamin E | mg | 0̸.22 | stearic acid | % | 75.5 |
| vitamin B2 | mg | 0̸.0̸7 | oleic acid | % | 0̸.9 |
| vitamin pp | mg | 0̸.81 | | | |

### Example 2

The tank used in example 1 was used. The fermentation medium was a 15% soybean milk medium (by weight of soybean ) consisting of850̸g yeart extract, 110̸0̸g KH₂PO₄, 250̸g MgSO₄, 4gCaCo₃, 430̸g NaCl, 30̸gNa₂Mo0̸₄, 5g Na₂SeO₃ , 15g ZnSo₄ , 15 CoCl₂ , 1kg Sodium benzoate and water. The inoculum was 1% consisted of YZH pseudomonas added in two stages as a first , second seed culture for about 50̸ hours. The inoculum was cultured at a temperature of 27°C , stirring speed of 20̸0̸ rmp and areation rate of 1 :1 v/v min (mass/air ). After the seed culture was finished , the inoculum was added in the 5 ton tank for a third continuous incubation. The fermentation medium and inoculum were allowed to react in the fermentation tank for a period of about 65 hours. The resulting cultured broth was autoclaved and harvested at 110̸°C and barometric pressure for about one hour and autoclaved again at 120̸°C, and the pressure known by the person skilled in the art for 2 hours to give a final product, that is our nutrient solution.

The features disclosed in the foregoing description, in the claims and/or in the accompanying drawings may, both separately and in any combination thereof, be material for realising the invention in diverse forms thereof.

## Claims

1. A mutant strain of pseudomonas identified as YZH and capable of converting plant protein to single cell protein, said strain being a blunt rod in light yellow colour, having at least one flagellum, a chemosynthetic heterotroph, obligatorily aerobic, not able to produce water soluble pigment, and to form intracellular polyhydroxy-butyrate, and to produce arginine dihydrolase, and to have no action of denitrification, and able to reduce nitrates and to liquidize gelatine, the growth temperature being about 10 - 41°C, the preferable pH being 4.5 - 9.0, the content of guanine and cytosine in DNA of the strain being 65.4 - 67.7 mol%, and the DNA of the strain containing a Y fragment and said strain having identifying characteristics of the sample on deposit with the American Type culture collection, 12301 Parklaw Drive, Rockville, Maryland, 20852, and asigned A.T.C.C. Deposit No. 53980.

2. The mutant strain of pseudomonas in accordance with claim 1, wherein said Y fragment in the DNA of the strain is detected by a special Y fragment DNA probe.

3. A process for producing a single cell protein nutrient solution by using the mutant strain of pseudomonas as claimed in claim 1 as a solution production strain and plant protein containing medium being used in a fermentation procedure to produce said nutrient solution.

4. The process in accordance with claim 3, wherein said plant protein containing medium is an aqueous medium comprising (by weight):
| Soybean medium | |
|---|---|
| Soybean | 5 - 10 % |
| or Soybean milk or bean cake (by weight of Soybean) | 5 - 15 % |
| or yeast powder | 0.2 - 0.5 % |
| or peptone, beef extract | 0.02 - 0.3 % |
| CaCO₃ | 0.05 - 0.25 % |
| KH₂PO₄ | 0.02 - 0.1 % |
| MgSO₄ | 0.01 - 0.05 % |
| NaCl | 0.01 - 0.04 % |
| Na₂MoO₄ | 5.0 - 30 ppm |
| Na₂SeO₃ | 2.5 - 15 ppm |
| ZnSO₄ | 2.5 - 40 ppm |
| CoCl₂ | 5.0 - 20 ppm. |

5. A nutrient solution comprising an aqueous solution of single cell protein and trace elements, said nutrient solution being obtainable by fermentation of a plant protein containing medium described in claim 4, with the mutant strain of pseudomonas as claimed in claim 1.

6. The nutrient solution in accordance with claim 5 for use as a medicament having an anti-aging effect in mammalian.

7. The nutrient solution in accordance with claim 5 for use as a medicament having the effect of stimulating and improving hemogenesis in mammals.

8. The nutrient solution in accordance with claim 5 for use as a medicament having the therapeutic effect on stomatic disorder in mammalian and human.

9. The nutrient solution in accordance with claim 5 for use as a medicament having the effect of inhibiting cancer cells growth and reversing the cancer cells into normal cells in mammalian and human.

10. The nutrient solution in accordance with claim 5 for use as a medicament having the effect of therapeutic effect on acute bleeding of the gastric mucosa in mammalian.

11. A product containing the nutrient solution as claimed in claim 5 or the mutant strain of pseudomonas as claimed in claim 1 as additives or replacements in human or animal foods or drugs.

## Patentansprüche

1. Ein Mutantenstamm von Pseudomonas, als YZH identifiziert und dazu in der Lage, Pflanzenprotein in einzelliges Protein umzuwandeln, wobei der Stamm eine stumpfe Stange in hellgelber Farbe ist, mit wenigstens einem Flagellum, einem chemosynthetisch heterotrophen Aerobionten, nicht dazu fähig, wasserlösliches Pigment zu erzeugen und intrazellulares Polyhydroxy-butyrat zu bilden und Arginin-Dihydrolase zu erzeugen und ohne denitrifizierende Wirkung und in der Lage, Nitrate zu reduzieren und Gelantine zu verflüssigen, wobei die Wachstumstemperatur etwa 10 - 41°C liegt, wobei der bevorzugte pH-Wert 4,5 - 9,0 ist, wobei der Gehalt an Guanin und Cytosin in der DNA des Stammes 65,4 - 67,7 mol% beträgt und die DNA des Stammes ein Y-Fragment enthält und wobei der Stamm indentifizierbare Eigenschaften der Probe hat, die bei der American Type Culture Collection, 12301 Parklaw Drive, Rockville, Maryland, 20852 hinterlegt worden ist und der die A.T.C.C. Hinterlegungsnummer 53980 zugeordnet ist.

2. Der Mutantenstamm von Pseudomonas gemäß Anspruch 1, wobei das Y-Fragment in der DNA des Stranges durch eine spezielle Y-Fragment-DNA-Sonde nachgewiesen wird.

3. Verfahren zum Herstellen einer Einzelzellen-Protein-Nährlösung durch Verwenden des Mutantenstammes von Pseudomonas, wie nach Anspruch 1 beansprucht, als ein Lösungs-Erzeugerstamm, und in dem das Pflanzenprotein enthaltende Medium in einer Fermentationsprozedur benutzt wird, um die Nährlösung zu erzeugen.

4. Verfahren nach Anspruch 3, bei dem das Pflanzenprotein enthaltende Medium ein wässriges Medium ist, das (bezogen auf das Gewicht) aufweist:
| Sojabohnenmedium | |
|---|---|
| Sojabohnen | 5 - 15 % |
| oder Sojabohnenmilch oder Bohnenkuchen (bezogen auf das Gewicht der Sojabohnen) | 5 - 15 % |
| oder Hefepulver | 0,2 - 0,5 % |
| oder Pepton, Fleischextrakt | 0,02 - 0,3 % |
| CaCO₃ | 0,05 - 0,25 % |
| KH₂PO₄ | 0,02 - 0,1 % |
| MgSO₄ | 0,01 - 0,05 % |
| NaCl | 0,01 - 0,04 % |
| Na₂MoO₄ | 5,0 - 30 ppm |
| Na₂SeO₃ | 2,5 - 15 ppm |
| ZnSO₄ | 2,5 - 40 ppm |
| CoCl₂ | 5,0 - 20 ppm. |

5. Nährlösung, mit einer wässrigen Lösung aus einzelligem Protein und Spurenelementen, wobei die Nährlösung durch Fermentation eines Pflanzenprotein enthaltenden Mediums erhalten wird, wie es in Anspruch 4 beschrieben ist, mit dem Mutantenstamm von Pseudomonas, wie in Anspruch 1 beansprucht.

6. Nährlösung nach Anspruch 5 zur Verwendung als ein Medikament mit einer Anti-Alterungswirkung bei Säugetieren.

7. Nährlösung nach Anspruch 5 zur Verwendung als ein Medikament mit der Wirkung des Anregens und Verbesserns der Blutbildung bei Säugetieren.

8. Nährlösung nach Anspruch 5 zur Verwendung als ein Medikament mit der therapeutischen Wirkung auf stomatische Fehlordnungen bei Säugetieren und Menschen.

9. Nährlösung nach Anspruch 5 zur Verwendung als ein Medikament mit der Wirkung des Hemmens des Krebszellenwachstums und des Umwandelns der Krebszellen in normale Zellen bei Säugetieren und Menschen.

10. Nährlösung nach Anspruch 5 zur Verwendung als ein Medikament mit der Wirkung des therapeutischen Effekts bei akuter Blutung der Magenschleimhaut bei Säugetieren.

11. Produkt, das die Nährlösung nach Anspruch 5 oder den Mutantenstamm von Pseudomonas nach Anspruch 1 enthält, als Additive oder Ersatznahrungen bei Nahrungsmitteln oder Drogen für Menschen oder Tiere.

## Revendications

1. Souche mutante du genre *Pseudomonas* identifiée par YZH et capable de convertir une protéine végétale en protéine de monocellules, ladite souche étant un bâtonnet obtus de couleur jaune clair, possédant au moins un flagelle, hétérotrophe à chimiosynthèse, aérobie strict, incapable de produire un pigment hydrosoluble, ni de former du polyhydroxybutyrate intracellulaire, ni de produire de l'arginine-dihydrolase, ni d'exercer une action de dénitrification, et capable de réduire les nitrates et de liquéfier la gélatine, la température de croissance étant d'environ 10 à 41°C, le pH préférable étant de 4,5 à 9,0, la teneur en guanine et cytosine de l'ADN de la souche étant de 65,4 à 67,7 mol %, et l'ADN de la souche contenant un fragment Y, et ladite souche ayant les caractéristiques d'identification de l'échantillon en dépôt à American Type Culture Collection, 12301 Parklaw Drive, Rockville, Maryland, 20852, et ayant reçu le N° de dépôt A.T.C.C. 53980.

2. Souche mutante du genre *Pseudomonas* selon la revendication 1, dans laquelle ledit fragment Y de l'ADN de la souche est détecté par une sonde d'ADN de fragment Y spéciale.

3. Procédé de production d'une solution nutritive de protéine de monocellules par utilisation de la souche mutante du genre *Pseudomonas* telle que revendiquée dans la revendication 1 comme souche productrice de solution et d'un milieu contenant une protéine végétale qui sont utilisés dans une opération de fermentation pour produire ladite solution nutritive.

4. Procédé selon la revendication 3, dans lequel ledit milieu contenant une protéine végétale est un milieu aqueux comprenant (en poids) :
| Milieu au soja | |
|---|---|
| soja | 5 - 10 % |
| ou lait de soja ou tourteau de soja (en poids de soja) | 5 - 15 % |
| ou poudre de levure | 0,2 - 0,5 % |
| ou peptone, extrait de boeuf | 0,02 - 0,3 % |
| CaCO₃ | 0,05 - 0,25 % |
| KH₂PO₄ | 0,02 - 0,1 % |
| MgSO₄ | 0,01 - 0,05 % |
| NaCl | 0,01 - 0,04 % |
| Na₂MoO₄ | 5,0 - 30 ppm |
| Na₂SeO₃ | 2,5 - 15 ppm |
| ZnSO₄ | 2,5 - 40 ppm |
| CoCl₂ | 5,0 - 20 ppm |

5. Solution nutritive comprenant une solution aqueuse de protéine de monocellules et d'oligo-éléments, ladite solution nutritive pouvant être obtenue par fermentation d'un milieu contenant une protéine végétale décrit dans la revendication 4 avec la souche mutante du genre *Pseudomonas* telle que revendiquée dans la revendication 1.

6. Solution nutritive selon la revendication 5, pour son utilisation comme médicament ayant un effet anti-vieillissement chez les mammifères.

7. Solution nutritive selon la revendication 5, pour son utilisation comme médicament ayant l'effet de stimuler et d'améliorer l'hématopoïèse chez les mammifères.

8. Solution nutritive selon la revendication 5, pour son utilisation comme médicament ayant un effet thérapeutique sur les affections gastriques chez les mammifères et les êtres humains.

9. Solution nutritive selon la revendication 5, pour son utilisation comme médicament ayant l'effet d'inhiber la croissance de cellules cancéreuses et de retransformer les cellules cancéreuses en cellules normales chez les mammifères et les êtres humains.

10. Solution nutritive selon la revendication 5, pour son utilisation comme médicament ayant un effet thérapeutique sur l'hémorragie aiguë de la muqueuse gastrique chez les mammifères.

11. Produit contenant la solution nutritive telle que revendiquée dans la revendication 5 ou la souche mutante du genre *Pseudomonas* telle que revendiquée dans la revendication 1 comme additifs ou succédanés dans des aliments ou médicaments pour animaux ou êtres humains.
